(19) Europäisches Patentamt
European Patent Office
Office européen des brevets

(11) Veröffentlichungsnummer: **0 075 906**
**A2**

(12) # EUROPÄISCHE PATENTANMELDUNG

(21) Anmeldenummer: 82108891.1

(22) Anmeldetag: 25.09.82

(51) Int.Cl.[3]: **C 11 D 1/92**

(30) Priorität: 30.09.81 DE 3138770

(43) Veröffentlichungstag der Anmeldung:
06.04.83 Patentblatt 83/14

(84) Benannte Vertragsstaaten:
BE CH DE FR GB IT LI NL

(71) Anmelder: HOECHST AKTIENGESELLSCHAFT
Postfach 80 03 20
D-6230 Frankfurt/Main 80(DE)

(72) Erfinder: Ehrl, Winfried, Dr.
Stethaimer Strasse 4
D-8262 Neuötting(DE)

(72) Erfinder: Reng, Alwin
Im Schulzehnten 22
D-6233 Kelkheim (Taunus)(DE)

(72) Erfinder: Quack, Jochen Meinhard
Wilhelm-Reuter-Strasse 16
D-6239 Eppstein/Taunus(DE)

(54) **Haar- und Körperreinigungsmittel mit einem Gehalt an Alkylsulfatobetainen.**

(57) Haar- und Körperreinigungsmittel mit einem Gehalt an Alkylsulfatobetain der Formel

$$R_1-\underset{\underset{R_3}{|}}{\overset{\overset{R_2}{|}}{N}}{}^{\oplus}-(CH_2CH_2O)_nSO_3^{(-)}$$

worin $R_1$ $C_8$-$C_{22}$-Alkyl oder Alkenyl, bevorzugt $C_{12}$-$C_{18}$-Alkyl, $R_2$ $C_1$-$C_4$-Alkyl, vorzugsweise Methyl, $R_3$ $C_1$-$C_4$-Hydroxialkyl oder eine Gruppe der Formel $-(CH_2CH_2O)_nH$ und n eine Zahl von 1 bis 10 bedeutet.

EP 0 075 906 A2

Croydon Printing Company Ltd.

Haar- und Körperreinigungsmittel mit einem Gehalt an Alkylsulfatobetainen

Die Reinigung der menschlischen Haut und Haare erfolgt im allgemeinen mit wäßrigen tensidhaltigen Lösungen. Als Tenside werden dabei anionische, kationische, amphotere oder nichtionische Verbindungen allein oder in Kombination benutzt. Beispielsweise werden häufig Seifen, Alkylsulfate, sekundäre Alkansulfonate, Alkylethersulfate, $\alpha$-Olefinsulfonate, Aminoxide, Acylaminpolyglykolethersulfate, Fettsäurekondensationsprodukte, Sulfobernsteinsäureester, Sarcoside, Fettalkoholpolyglykolether zur Reinigung benutzt. Diese Reinigungsmittel haben die primäre Aufgabe, körperfremde Verunreinigungen und überschüssigen Hauttalg von den Körper- und Haaroberflächen zu entfernen. Als Indikator für den Ablauf des Reinigungsvorganges dient die durch Mechanik erzeugte Schaummenge bei der Anwendung der wäßrigen Tenside während des Reinigungsprozesses. Je nach verwendetem Tensidtyp ist die Art und Menge des erzielten Schaumes sehr unterschiedlich. Das gleiche gilt auch für den jeweils erzielbaren Reinigungseffekt. Beispielsweise ergeben anionische Tenside, wie Alkylsulfate, Alkylbenzolsulfonate oder Seifen bei der Anwendung einen sehr starken Reinigungseffekt, der beim Verbraucher nach der Anwendung das Gefühl der "ausgetrockneten" Hautoberfläche hervorruft und speziell bei Haarwaschmitteln die Kämmbarkeit des gewaschenen Haares wesentlich erschwert.

Es wurde deshalb versucht, durch Zusätze von kationischen Polymeren, Alkylbetainen oder Alkylamidobetainen diese Nachteile zu vermindern. Während kationische polymere Substanzen durch eine geringe Wasserlöslichkeit und eine schwierige Synthese sowie sehr geringe Schaumeigenschaften charakterisiert sind, besitzen Alkylbetaine, wie z.B. Cocosdimethylbetain den Nachteil der schlechten Augenschleimhautverträglichkeit. Alkylamidobetaine hingegen weisen als Nebenprodukte einen hohen Natriumchloridgehalt

auf, der häufig zu Korrosionen bei der Lagerung bzw. Verarbeitung führen kann. Schließlich zeigen die vorerwähnten Tenside bei alleiniger Verwendung nur mittleres Schaumverhalten und ergeben in Kombination mit anderen Tensiden keine ausgeprägte Verbesserung der Naß- und Trockenkämmbarkeiteigenschaften von Humanhaaren.

Es wurde nun gefunden, daß die nachstehend beschriebenen Alkylsulfatobetaine diese Nachteile nicht besitzen und sich somit mit diesen Substanzen Haut- und Haarwaschmittel herstellen lassen, die durch sehr gute Schaumeigenschaften charakterisiert sind und außerdem den Griff der Haare und die Kämmbarkeit positiv beeinflussen. Außerdem zeigen die Alkylsulfatobetaine beispielsweise in Shampoos, Schaumbädern, Duschbädern oder Intimwaschmitteln oder anderen Hautwaschmitteln nicht den Nachteil des "klebrigen" Hautgefühls nach der Anwendung.

Gegenstand der vorliegenden Erfindung sind Haar- und Körperreinigungsmittel mit einem Gehalt an Alkylsulfatobetain der Formel

$$R_1 - \overset{\oplus}{\underset{\substack{| \\ R_3}}{\overset{\substack{R_2 \\ |}}{N}}} - (CH_2CH_2O)_nSO_3^{\ominus}$$

worin $R_1$ $C_8$-$C_{22}$-Alkyl oder Alkenyl, bevorzugt $C_{12}$-$C_{18}$-Alkyl, $R_2$ $C_1$-$C_4$-Alkyl, vorzugsweise Methyl, $R_3$ $C_1$-$C_4$-Hydroxialkyl oder eine Gruppe der Formel $-(CH_2CH_2O)_nH$ und n eine Zahl von 1 bis 10 bedeutet.

Die Sulfobetaine der obigen Formel werden hergestellt nach bekannten Verfahren, indem man ein Amin der Formel

$$R_1 - \underset{\substack{| \\ R_3}}{N} - (CH_2CH_2O)_nH$$

zunächst unter Einführung des Restes $R_2$ quaternisiert und die so erhaltene quaternäre Ammoniumverbindung dann sulfatiert. Als Quaternierungsmittel kommen die für diese Reaktion bekannten Verbindungen in Frage wie Alkylhalogenide, Alkylschwefelsäureester oder Alkylphosphorsäureester. Bevorzugt sind Methylchlorid und Dimethylsulfat. Die Reaktion wird in einem geeigneten Lösemittel durchgeführt bei dessen Siedetemperatur. Geeignete Lösemittel sind z.B. niedere Alkohole und Acetonitril.

Die Sulfatierung erfolgt anschließend im gleichen oder in einem anderen Lösemittel bei Temperaturen von 0 bis 60°C, vorzugsweise bei 30°C. Geeignete Lösemittel für diese Reaktion sind außer Acetonitril auch halogenierte Kohlenwasserstoffe. Als Sulfatierungsmittel nimmt man beispielsweise konzentrierte Schwefelsäure, Lösungen von Schwefeltrioxid in Schwefelsäure, sowie insbesondere Chlorsulfonsäure und Schwefeltrioxid. Die Menge an Sulfatierungsmittel wird so gewählt, daß in das quaternierte Amin nur eine Sulfatogruppe eingeführt wird.

Die nach den vorstehenden Verfahren synthetisierten Alkylsulfatobetaine lassen sich problemlos zur Herstellung von Haar- und Körperreinigungsmitteln verwenden. Die Tenside können allein oder in Kombination mit anderen üblicherweise verwendeten anionischen, kationischen, amphoteren und/oder nichtionischen Tensiden verarbeitet werden. Sie lassen sich beispielsweise in Haarshampoos, Schaumbädern, Gesichtswaschmitteln, Duschbädern, Hand- und Fußwaschmitteln, Intimwaschmitteln und anderen speziellen kosmetischen Reinigungsmitteln einarbeiten.

Die Alkylsulfatobetaine sind mit den meisten der für diese kosmetischen Reinigungsmittel verwendeten Komponenten gut verträglich. Dies können beispielsweise viskositätserhöhende Substanzen, wie Celluloseether, Elektrolyte, wie Natriumchlorid, Magnesiumchlorid, Ammoniumchlorid, oder Magnesiumaluminiumsilikate, hochdisperse, amorphe Kieselsäure, Polyacrylamide oder ähnliche Substanzen sein. Auch Wirkstoffe, wie z.B. Allantoin, Antischuppenwirkstoffe, Konservierungsmittel, UV-Absorber, Farbstoffe können in die Alkylsulfatobetain-haltigen Reinigungspräparate eingearbeitet werden. Je nach Anwendungszweck können die Reinigungsmittel pulverförmig, gelförmig oder flüssig hergestellt werden. Bei der Herstellung der flüssigen Produkte können neben Wasser noch weitere flüssige Bestandteile wie 1,2-Propylenglycol, Polyethylenglycole, Glycerin, Ethanol zugesetzt werden. Der pH-Wert dieser Reinigungsmittel kann je nach Anwendungszweck zwischen 4 und 9 eingestellt werden.

Die Einsatzmengen der Alkylsulfatobetaine liegen im allgemeinen zwischen 1 und 50 %, vorzugsweise jedoch zwischen 5 und 20 %, bezogen auf das Gewicht der fertigen Formulierung. Es ist auch möglich, die Alkylsulfatobetaine mit anderen, üblicherweise in kosmetischen Reinigungsmitteln eingesetzten grenzflächenaktiven Substanzen, wie z.B. Alkylsulfaten, Alkylethersulfaten, Seifen, Carbonsäuresarcosiden, Carbonsäureeiweißkondensationsprodukten, Sulfobernsteinsäureestern, α-Olefinsulfonaten, sekundären Alkansulfonaten sowie mit nichtionischen Alkoholpolyglykolethern - oder Estern zu kombinieren.

Beispiel 1

In einem 1-Liter Glasautoklaven werden 447 g (1,5 mol) N,N-Bis-hydroxyethyl-cocosfett-alkylamin und 300 ml Acetonitril eingegeben. Dann drückt man die benötigte Menge Methylchlorid in Portionen bei 80°C auf. Die Reaktion ist praktisch beendet, wenn kein Methylchlorid mehr aufgenommen wird. Das Reaktionsprodukt enthält 97,3 % der quaternierten Verbindung.

Dieses Reaktionsprodukt wird mit weiteren 500 ml Acetonitril verdünnt in einen 4-Liter Dreihalskolben mit Rührer, Tropftrichter, Rückflußkühler und Thermometer eingebracht. Dann setzt man unter Rühren 175 g (1,5 mol) Chlorsulfonsäure so zu, daß die Temperatur im Reaktionsgefäß 30°C nicht übersteigt. Danach erhitzt man den Ansatz eine Stunde unter Rühren am Rückfluß. Man destilliert anschließend das Lösemittel ab und nimmt den Rückstand mit Wasser auf zu einer 30 %igen Lösung der Verbindung der Formel

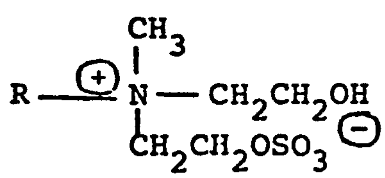

R = Cocosfettalkyl.

Beispiel 2

In einen 2-Liter Dreihalskolben mit Rührer, Tropftrichter, Rückflußkühler und Thermometer gibt man 745 g (2,5 mol) N,N-Bis-hydroxyethyl-cocosfett-alkylamin und löst in 500 ml Isopropanol. Bei 50 - 60°C werden dann unter Rühren 320 g Dimethylsulfat (2,54 mol) langsam zugetropft. Man läßt das Reaktionsgemisch 2 Stunden bei 60°C nachreagieren. Anschließend wird das Lösemittel am Rotationsverdampfer bei 60°C und im Wasserstrahlvakuum abgezogen. Der quaternäre Anteil wurde mit 97 % ermittelt. Der Trockenrückstand aus der Quaternisierung wird in 1000 ml Acetonitril aufgenommen

und in einen 4-Liter Dreihalskolben mit Tropftrichter, Rühter, Rückflußkühler und Thermometer eingebracht. Dann setzt man unter Rühren 295 g (2,53 mol) Chlorsulfonsäure so zu, daß die Temperatur im Reaktionsgefäß 30°C nicht übersteigt. Danach erhitzt man den Ansatz eine Stunde unter Rühren am Rückfluß, destilliert anschließend das Lösemittel am Rotationsverdampfer ab und nimmt den Rückstand mit Wasser auf zu einer 30 %igen Lösung der Verbindung der Formel

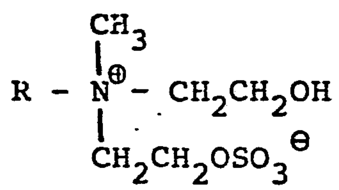

R = Cocosfettalkyl.

Die Lösung wird mit Natronlauge auf den pH-Wert 7 eingestellt. Durch Bleichen mit $H_2O_2$ (2 % berechnet auf den Feststoff) erreicht man eine Aufhellung der Lösung von Jod-Farbzahl 3 nach 1.

Analysenergebnis:

Freies $SO_4^{--}$ = 0,4 %

Hydrolysierbares $SO_4^{--}$ 9,8 %

Beispiel 3

In einen 1-Liter Dreihalskolben mit Rührer, Tropftrichter, Rückflußkühler und Thermometer gibt man 146 g (0,5 mol) N,N-Bis-hydroxyethyl-cocosfett-alkylamin, löst in 200 ml Acetonitril und tropft unter Rühren bei 50 - 60°C 63 g (0,5 mol) Dimethylsulfat zu. Man läßt dann noch eine Stunde bei 60°C nachreagieren.

Zu dieser Reaktionsmischung aus der Quaternisierung tropft man langsam, die Temperatur im Reaktionsgefäß soll 30°C nicht übersteigen, 58,3 g (0,5 mol) Chlorsulfonsäure zu und läßt anschließend noch zwei Stunden bei 30°C nachreagieren. Dabei wird ein schwaches Vakuum angelegt, um das restliche HCl-Gas zu entfernen.

Anschließend dampft man das Lösemittel am Rotationsverdampfer ab. Der Trockenrückstand wird mit Wasser zu einer 30 %igen Lösung aufgenommen und mit Natronlauge auf einen pH-Wert 7 eingestellt. Durch Bleichen mit $H_2O_2$ (2 % berechnet auf den Feststoff) erreicht man eine Aufhellung der Lösung auf eine Jod-Farbzahl von 4 nach 1. Das so erhaltene Sulfobetain hat die Formel

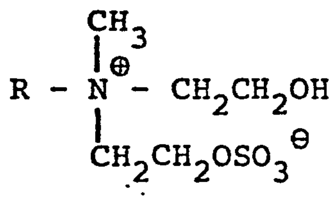

R = Cocosfettalkyl.

Beispiel 4

In einen 1-Liter Dreihalskolben mit Rührer, Tropftrichter, Rückflußkühler und Thermometer gibt man 422 g (1 mol) N,N-Bis-pentaoxyethylen-cocosfettalkylamin in 400 ml Acetonitril, tropft unter Rühren 126 g (1 mol) Dimethylsulfat bei 50 - 60°C zu und läßt eine Stunde bei 60°C nachreagieren. Zu der Reaktionsmischung aus der Quaternisierung tropft man dann langsam, die Temperatur im Reaktionsgefäß soll dabei nicht über 30°C ansteigen, 116,5 g (1 mol) Chlorsulfonsäure zu und läßt zwei Stunden bei 60°C nachreagieren. Dabei setzt man ein schwaches Vakuum an, um das restliche HCl-Gas zu entfernen. Anschließend wird das Lösemittel am Rotationsverdampfer abgezogen. Der Trockenrückstand wird mit Wasser zu einer 30 %igen Lösung aufgenommen und mit Natronlauge auf einen pH-Wert 7 eingestellt. Durch Bleichen mit $H_2O_2$ (2 % berechnet auf den Feststoff) erreicht man eine Aufhellung von Jod-Farbzahl 20 auf 3. Das so erhaltene Sulfobetain hat die Formel

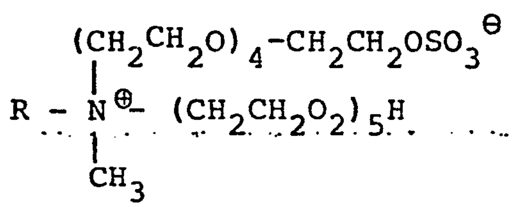

R = Cocosfettalkyl.

Beispiel 5

In einen 1-Liter Dreihalskolben mit Rührer, Tropftrichter, Rückflußkühler und Thermometer gibt man 500 g (0,78 mol) N,N-Bis-decaoxyethylen-cocosfettalkylamin, löst in 400 ml Acetonitril und tropft unter Rühren bei 50 - 60°C 98 g (0,78 mol) Dimethylsulfat zu. Man läßt das Reaktionsgemisch noch eine Stunde bei 60°C nachreagieren. Zu der Reaktionsmischung aus der Quaternisierung tropft man langsam, die Temperatur im Reaktionsgefäß soll dabei nicht über 30°C ansteigen, 90,3 g (0,78 mol) Chlorsulfonsäure zu und läßt anschließend bei 30°C zwei Stunden lang nachreagieren. Das entstandene HCl-Gas wird dabei unter leicht vermindertem Druck entfernt. Anschließend wird das Lösemittel am Rotationsverdampfer abgezogen. Der Trockenrückstand wird in Wasser zu einer 30 %igen Lösung aufgenommen und mit Natronlauge auf einen pH-Wert 7 eingestellt. Durch Bleichen mit $H_2O_2$ (2 % berechnet auf den Feststoff) erreicht man eine Aufhellung der Lösung von Jod-Farbzahl 8 nach 3. Das so erhaltene Sulfobetain hat die Formel

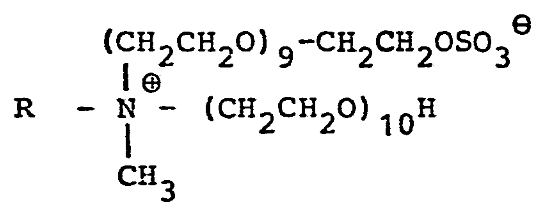

R = Cocosfettalkyl.

Beispiel 6

In einen 1-Liter Dreihalskolben mit Rührer, Tropftrichter, Rückflußkühler und Thermometer gibt man 460 g (1,28 mol) N,N,-Bis-hydroxyethyl-cocosfettalkylamin, löst in 400 mol Acetonitril und tropft unter Rühren 161,3 g (1,28 mol) Dimethylsulfat zu bei 50 - 60°C. Zu der Reaktionsmischung aus der Quaternisierung tropft man langsam, die Temperatur im Reaktionsgefäß soll dabei nicht über 30°C ansteigen, 149,1 g (1,28 mol) Chlorsulfonsäure zu. Man läßt 2 Stunden bei 30°C nachreagieren, wobei das restliche HCl-Gas durch ein schwaches Vakuum entfernt wird. Anschließend wird das

Lösemittel am Rotationsverdampfer abgezogen. Der Trockenrückstand wird in Wasser zu einer 30 %igen Lösung aufgenommen und mit Natronlauge auf einen pH-Wert 7 eingestellt. Durch Bleichen mit $H_2O_2$ (2 % berechnet auf den Feststoff) erreicht man eine Aufhellung der Lösung von Jod-Farbzahl 12 nach 2. Man erhält so ein Sulfobetain der Formel

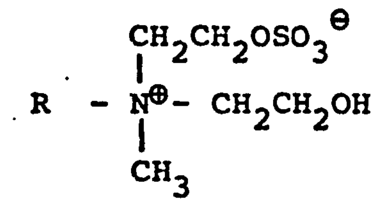

R = Cocosfettalkyl.

Die nachstehenden Prüfungen lassen die Vorteile, die man beim Einsatz von Alkylsulfatobetainen in kosmetischen Körper- und Haarreinigungsmitteln erhält, erkennen:

1.) Schaumvermögen

Das Schaumvermögen wurde in Anlehnung an die Methode nach ROSS-MILES (S.J. Gohlke, "Die Bestimmung des Schaumvermögens von Detergentien nach ROSS-MILES" Parfümerie und Kosmetik 45, 359 - 63 (1964) in Wasser von 20° dH bei einer Temperatur von + 43° C bestimmt. Als Vergleichssubstanz diente ein Fettsäureamidoalkylbetain folgender Zusammensetzung:

$$R\text{-}CO\text{-}NH\text{-}CH_2CH_2CH_2\text{-}\overset{\displaystyle CH_3}{\underset{\displaystyle CH_3}{N^{(+)}}}\text{-}CH_2\text{-}COO^{(-)}$$

$R = C_{12/14}$

Dieser Betaintyp wurde mit einem Alkylsulfatobetain der Formel

$$R - \overset{\displaystyle CH_2CH_2OH}{\underset{\displaystyle CH^3}{N^{(+)}}} -(CH_2\ CH_2O)-SO_3{}^{(-)}$$

$R = C_{12/14}$

verglichen. Dabei wurden die in der folgenden Tabelle angegebenen Schaumwerte gemessen. In beiden Fällen ist das Schaumverhalten des erfindungsgemäß zu verwendenden Alkylsulfatobetains besser.

 

| Konzentration in % | Schaumhöhe in mm Alkylsulfatobetain | Alkylamidobetain |
|---|---|---|
| 0,002 | 30 | 20 |
| 0,006 | 60 | 50 |
| 0,03 | 150 | 135 |
| 0,1 | 210 | 180 |
| 0,3 | 240 | 200 |
| 1 | 250 | 225 |

2. Hautgefühl

Die Prüfung des "Hautgefühls" nach dem Reinigungsprozeß wurde nach folgender Methode durchgeführt: Jeweils 10 g Tensid wurden unter Rühren in 90 g destilliertem Wasser gelöst. Mit 20 g dieser Lösung wuschen sich 10 neutrale Versuchspersonen 25 sec lang die Hände und spülten mit 300 ml Leitungswasser von 35° C nach. Anschließend wurden die Hände mit einem sauberen Baumwollhandtuch getrocknet und das vorhandene Hautgefühl nach dem Abtrocknen subjektiv wiedergegeben. Die Beurteilungen brachten folgende Ergebnisse:

| | |
|---|---|
| Alkylsulfatobetain: | weiches, angenehmes Hautgefühl gemäß Beispiel 1 |
| Natriumlautylsulfat: | sehr klebriges unangenehmes Hautgefühl |
| Alkyldiglycolether-sulfat-Natriumsalz: | klebriges, unangenehmes Hautgefühl |

Nach 5 Minuten erfolgte eine Wiederholung der Beurteilung mit folgendem Ergebnis:

| | |
|---|---|
| Alkylsulfatobetain: | weiches, angenehmes Hautgefühl |
| Natriumlaurylsulfat: | trockene, entfettete Haut, die "spannt". |
| Alkyldiglycolether-sulfat-Natriumsalz: | trockenes, rauhes Hautgefühl, leicht "spannend" |

3. Haar-Kämmbarkeit

Es wurden 15 cm lange mitteleuropäische Haarsträhnen mit einem Durchmesser von 1,5 cm jeweils mit der zu testenden, 15 % Tensid enthaltenden wäßrigen Lösung gewaschen und anschließend nach dem Spülen mit Leitungswasser von 35°C sowohl die Kämmbarkeit des nassen Haares als auch der Griff und die Beschaffenheit der Haare beurteilt. Die Ergebnisse sind aus der nachstehenden Tabelle zu ersehen.

| Tensid | Naßkämmbarkeit | Griff der Haare | Trockenkämmbarkeit |
|---|---|---|---|
| Alkylsulfato-betain gemäß Beispiel 1 | sehr gut | weich, glatt | sehr gut |
| Laurylsulfat-Natriumsalz | sehr schlecht | hart, rauh | schlecht |
| Lauryldiglykol-ethersulfat-Natriumsalz | schlecht | hart, rauh | mäßig |

4. Physikalische und chemische Lagerstabilität

Proben einer 1%igen wäßrigen Lösung der Verbindungen der Beispiele 1 - 6 wurden 5 Monate bei +25°C und +40° C gelagert. Nach jeweils 14 Tagen wurde elektrometrisch der pH-Wert und die Jodfarbzahlen, nach DIN 6162, gemessen. Es konnten keine gravierenden Änderungen des pH-Wertes und der Farbe festgestellt werden.

Die nachfolgend aufgeführten Beispiele sollen die Einsatzmöglichkeiten der Alkylsulfatobetaine in Haar- und Körperreinigungsmitteln veranschaulichen. Die Mengen und %-Angaben in den Beispielen beziehen sich, sofern nicht anders erwähnt, jeweils auf das Gewicht.

Beispiel 1

1. Haarspampoo mit konditionierendem Effekt

| | |
|---|---|
| Alkylsulfatobetain-Natriumsalz gemäß Beispiel 1 | 15,0 % |
| Hydroxyethylcellulose-Ether | 1,2 % |
| Parfümöl | 0,3 % |
| Formaldehyd | 0,05% |
| Wasser | ad 100 % |

2. Haarshampoo mit mildem Reinigungsvermögen

| | |
|---|---|
| Alkylsulfatobetain-Natriumsalz gemäß Beispiel 1 | 7,0 % |
| Acylaminopolyglycolethersulfat-triethanolaminsalz | 7,0 % |
| Cocosfettsäurediethanolamid | 2,0 % |
| Triethylenglykoldistearat | 1,0 % |
| Natriumchlorid | 2,0 % |
| Parfümöl | 0,3 % |
| Formaldehyd | 0,05% |
| Wasser | ad 100 |

3. saures Shampoo

| | |
|---|---|
| Alkylsulfatobetain-Natriumsalz gemäß Beispiel 1 | 10,0 % |
| Lauryldimethylaminoxid | 5,0 % |
| Zitronensäure | 0,4 % |
| Parfümöl | 0,2 % |
| Konservierungsmittel, Farbstoffe Wasser | ad 100 % |

4. Shampoo für trockene Haare

| | |
|---|---|
| Alkylsulfatobetain-Natriumsalz gemäß Beispiel 4 | 12,0 % |
| Pentaoxethylammoniumchlorid | 1,0 % |
| Alkyltriglycolethersulfat-Natriumsalz | 3,0 % |
| Ethylenglykolmonostearat | 0,7 % |
| Polyethylenglykol-6000-distearat | 4,0 % |
| Wasser, Konservierungsmittel, Farbstoffe | ad 100 % |

5. Antischuppenshampoo

| | |
|---|---|
| Alkylsulfatobetain-Natriumsalz gemäß Beispiel 5 | 12,0 % |
| Palmkernfettsäuremethyltaurid-Natriumsalz | 15,0 % |
| Stearinsäuremethyltaurid-Natriumsalz | 6,0 % |
| Lauroylsarkosid-Natriumsalz | 2,0 % |
| 2-Mercaptopyriden-N-Oxid-Zinksalz | 0,5 % |
| Parfümöl | 0,3 % |
| Wasser | ad 100 % |

6. Duschbad

| | |
|---|---|
| Alkylsulfatobetain gemäß Beispiel 1 | 10,0 % |
| Lauryltriglykolethersulfosuccinat-di-Natriumsalz | 5,0 % |

| | |
|---|---|
| Hydroxyethylcelluloseether | 1,0 % |
| Parfümöl | 0,2 % |
| Cocosfettsäure-mono-ethanolamid | 1,5 % |
| Imidazolidinyl-Harnstoff | 0,3 % |
| Wasser | ad 100 % |

7. Handwaschmittel

| | |
|---|---|
| Alkylsulfatobetain-Natriumsalz gemäß Beispiel 3 | 6,0 % |
| sekundäres Alkansulfonat-Natriumsalz | 4,0 % |
| Cocosfettsäure-isethionat-Natriumsalz | 3,0 % |
| Cocosfettsäure-diethanolamid | 2,0 % |
| 2,4,4'-Trichlor-2-hydroxydiphenylether | 0,1 % |
| Milchsäure | 0,15% |
| Wasser | ad 100 % |

8. Fußwaschmittel

| | |
|---|---|
| Alkylsulfatobetain gemäß Beispiel 1 | 8,0 % |
| Laurylsulfat-Natriumsalz | 3,0 % |
| Lauryldecaglykolether | 2,0 % |
| Undecylensäure-monoethanolamid | 3,0 % |
| Formaldehyd | 0,05% |
| Wasser | ad 100 % |

9. Intimwaschmittel

| | |
|---|---|
| Alkylsulfatobetain gemäß Beispiel 1 | 6,0 % |
| Cocosethylcycloimidino-1-hydroxy-3-ethylnatriumalkoholat-2-methylnatriumcarboxylat | 5,0 % |
| Zitronensäure | 0,3 % |
| Alkyltriglykolethersulfat-triethanolaminsalz | 3,0 % |

| | |
|---|---|
| 3,4,4'-Trichlorcarbanilid | 0,2 % |

10. Schaumbad

| | |
|---|---|
| Alkylsulfatbetain-Natriumsalz gemäß Beispiel 6 | 10,0 % |
| Cocosdiglykolethersulfat-Natriumsalz | 30,0 % |
| sek. Alkansulfonat | 10,0 % |
| Ölsäurediethanolamid | 1,0 % |
| Parfümöl | 1,0 % |
| Natriumchlorid | 2,5 % |
| Wasser | ad 100 % |

Patentanspruch

Haar- und Körperreinigungsmittel mit einem Gehalt an Alkylsulfatobetain der Formel

$$R_1-\overset{\displaystyle R_2}{\underset{\displaystyle R_3}{N^{\oplus}}}-(CH_2CH_2O)_nSO_3^{(-)}$$

worin $R_1$ $C_8$-$C_{22}$-Alkyl oder Alkenyl, bevorzugt $C_{12}$-$C_{18}$-Alkyl, $R_2$ $C_1$-$C_4$-Alkyl, vorzugsweise Methyl, $R_3$ $C_1$-$C_4$-Hydroxialkyl oder eine Gruppe der Formel $-(CH_2CH_2O)_nH$ und n eine Zahl von 1 bis 10 bedeutet.